# EUROPEAN PATENT APPLICATION

(11) **EP 4 592 292 A1**
(43) Date of publication of application: **30.07.2025**
(21) Application number: 23868345.2
(22) Date of filing: 17.07.2023
(51) Int. Cl.: C07D 487/08, A61K 31/551

(54) **STABLE MONOHYDRATE FORM OF AVIBACTAM SODIUM SALT AND PREPARATION METHOD THEREFOR**

(30) Priority: 23.09.2022 KR 20220120597
(71) Applicant: Kyongbo Pharm. Co., Ltd., Asan-si, Chungcheongnam-do 31501 (KR)
(72) Inventor: HWANG, Jae Taeg, Suwon-si Gyeonggi-do 16505 (KR); KIM, Byung Ok, Asan-si Chungcheongnam-do 31579 (KR); KIM, Hak Chun, Pyeongtaek-si Gyeonggi-do 17870 (KR); KIM, Su Ho, Asan-si Chungcheongnam-do 31525 (KR); KIM, Min Seok, Asan-si Chungcheongnam-do 31503 (KR); KIM, Chae Lin, Changwon-si Gyeongsangnam-do 51273 (KR); HEO, Hoon Gu, Seoul 05090 (KR)
(74) Representative: Turner, Craig Robert
(86) International application number: PCT/KR2023/010153
(87) International publication number: WO 2024/063285

(57) **Abstract**

The present invention relates to an industrially useful and pharmaceutically stable monohydrate of avibactam sodium salt and a preparation method therefor. The compound of the present invention can be used as a beta-lactamase inhibitor.

## Description

### TECHNICAL FIELD

The present invention relates to an industrially useful and pharmaceutically stable monohydrate of avibactam sodium salt and a process for preparing the same.

### BACKGROUND ART

WO2002/10172 describes azabicyclic compounds and salts thereof.

WO2003/063864 describes the use as a β-lactamase inhibitor and the use of said β-lactamase inhibitor in combination with β-lactamamine antibiotics such as ceftazidime.

WO2011/042560 A1 describes that (1R,2S,5R)-7-oxo-6-sulfooxy-1,6-diazabicyclo[3.2.1]octane-2-carboxamide sodium (avibactam sodium salt) of the chemical formula 1 exists in anhydrous forms "B", "D", in a monohydrate form "A", and in a dihydrate form "E" and that an anhydrous form "C" is observed only as a mixture with Form "A".

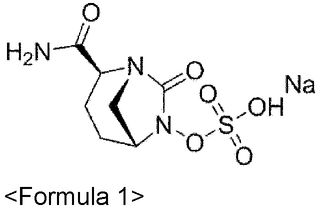

WO2011/042560 A1 describes that the anhydrous form "D" is very small crystals making filtration difficult and hence making it difficult to prepare Form D industrially; that the dihydrate form "E" tends to lose water and hydrolyze during long storage and at higher temperature; and that the anhydrous forms "B" and the monohydrate form "A" are preferable forms. However, the form "B" can be stably obtained when the form "A" is used as a seed and the form "A" can also be stably obtained when the form "A" is used as a seed. Therefore, it is considered that the form "A", the form "B", the form "D", and the form "E" are difficult to manufacture industrially.

### DETAILED DESCRIPTION OF THE INVENTION

### Technical Problem

The present invention is to solve the above problems and thus it is an object of the present invention to provide an industrially useful and pharmaceutically stable monohydrate of avibactam sodium salt and a process for preparing the same.

### Technical Solution

It has been found by the present invention that a novel monohydrate form of avibactam sodium salt (hereinafter referred to as form "α") can be prepared in an industrially efficient and effective manner, compared to the monohydrate form "A"; and that it is a pharmaceutically stable form.

More specifically, the compound of the present invention does not have filtration problems due to small crystals (e.g., the form "D"), can be prepared without using seeds (e.g., the form "B"), and is pharmaceutically more stable than the form "B". Additionally, unlike the forms "E" and "A", it is a pharmaceutically stable crystalline form.

In particular, it has been confirmed in the present invention that the form "α" of the present invention is a novel monohydrate form, which is a different form from the monohydrate form "A" described in WO2011/042560 A1, through physicochemical characterizations; and that the novel monohydrate form "α" of the present invention is a more stable form through the comparative accelerated stability test (40°C, 75% relative humidity). In addition, it has been confirmed that the form "α" maintained the monohydrate form thereof in the accelerated stability test (40°C, 75% relative humidity).

The present invention provides a novel crystalline monohydrate form "α" of avibactam sodium salt having an X-ray diffraction pattern comprising characteristic peaks at 10.0±0.5°, 15.0±0.5°, 16.0±0.5°, and 19.4±0.5° 2-theta (2θ).

More specifically, the present invention provides a novel crystalline monohydrate form "α" of avibactam sodium salt having an X-ray diffraction pattern comprising characteristic peaks at 10.0±0.5°, 13.2±0.5°, 14.2±0.5°, 15.0±0.5°, 16.0±0.5°, 18.0±0.5°, and 19.4±0.5° 2-theta (2θ).

The present invention also provides a process for preparing the novel crystalline monohydrate form "α". Specifically, the present invention provides a process for preparing a crystalline monohydrate of avibactam sodium salt, comprising (a) adding a solution of sodium 2-ethylhexanoate to a solution of avibactam tetrabutylammonium salt; (b) stirring the reaction mixture of step (a) at room temperature, followed by stirring at -10°C to -5°C to form a crystal; and (c) isolating the crystal.

Preferably, the process of the present invention does not comprise seeding a monohydrate of avibactam sodium salt.

In the process of the present invention, the solution of avibactam tetrabutylammonium salt may be a solution of avibactam tetrabutylammonium salt in a mixed solvent of C₁-C₄ alcohol and water. The mixed solvent of C₁-C₄ alcohol and water may preferably be a mixed solvent of methanol and water. The mixed solvent of methanol and water may be a mixed solvent having a volume ratio of 4 to 8 : 1, preferably about 5.5 : 1, of methanol : water.

In the process of the present invention, the solution of sodium 2-ethylhexanoate may be a solution of sodium 2-ethylhexanoate in C₁-C₄ alcohol or in a mixed solvent of C₁-C₄ alcohol and water. The C₁-C₄ alcohol or the mixed solvent of C₁-C₄ alcohol and water may be methanol or a mixed solvent of methanol and water, respectively.

In the process of the present invention, step (c) may be carried out by washing the crystal obtained by filtering the mixture of step (b) with acetone at a temperature of 0°C to 5°C, e.g., a temperature of about 5°C, followed by drying the resulting crystal.

### ADVANTAGEOUS EFFECTS

An industrially useful and pharmaceutically stable novel monohydrate form "α" of avibactam sodium salt, which can be used as a beta-lactamase inhibitor, is provided by the present invention.

### BRIEF DESCRIPTION OF THE DRAWINGS

FIG. 1 shows the results of the comparative accelerated stability test on purity (40°C, 75% relative humidity) for the novel monohydrate form "α" of the present invention, the monohydrate form "A" and the anhydrous form "B" described in WO2011/042560 A1.
FIG. 2 shows the results of the comparative accelerated stability test on appearance changes (40°C, 75% relative humidity) for the novel monohydrate form "α" of the present invention, the monohydrate form "A" and the anhydrous form "B" described in WO2011/042560 A1.
FIG. 3 shows the results of the accelerated stability test on moisture content changes (40°C, 75% relative humidity) for the novel monohydrate form "α" of the present invention.
FIG. 4 shows the XRD pattern of the novel monohydrate form "α" of the present invention.
FIG. 5 shows the TGA pattern of the novel monohydrate form "α" of the present invention.
FIG. 6 shows the DSC pattern of the novel monohydrate form "α" of the present invention.

### BEST MODE FOR CARRYING OUT THE INVENTION

The present invention is described by the following examples.

### Example 1: Preparation of the novel monohydrate form "α" of avibactam sodium

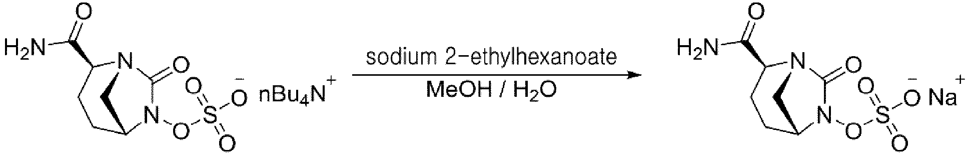

To a solution of avibactam tetrabutylammonium salt (17.64 g, 34.2 mmol) in a mixed solution of methanol (110 ml) and purified water (20 mL), was added a solution of sodium 2-ethylhexanoate (11.57 g, 69.6 mmol) in methanol (50 ml) over 45 minutes. The resulting reaction mixture was stirred at room temperature for about 1 hour and then stirred at about -10°C to -5°C overnight. After filtering the mixture, the obtained crystals were washed with acetone (50 ml) at 5°C and dried under vacuum to obtain a novel monohydrate form "α". (6.5 g, Yield 75.4%).

### Example 2. Comparative accelerated stability test on purity/appearance changes (40°C, 75% relative humidity)

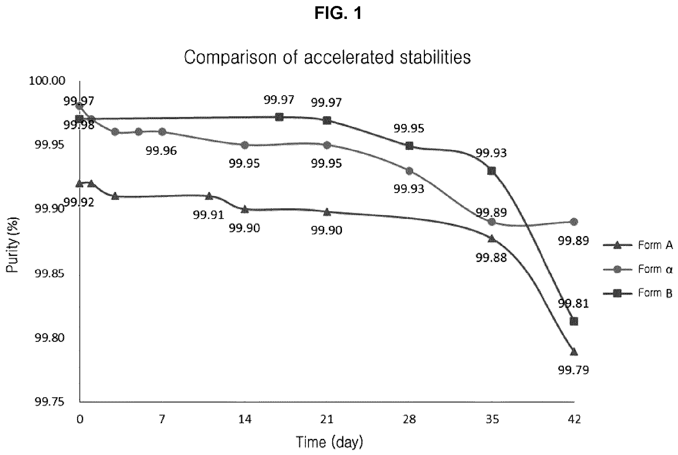

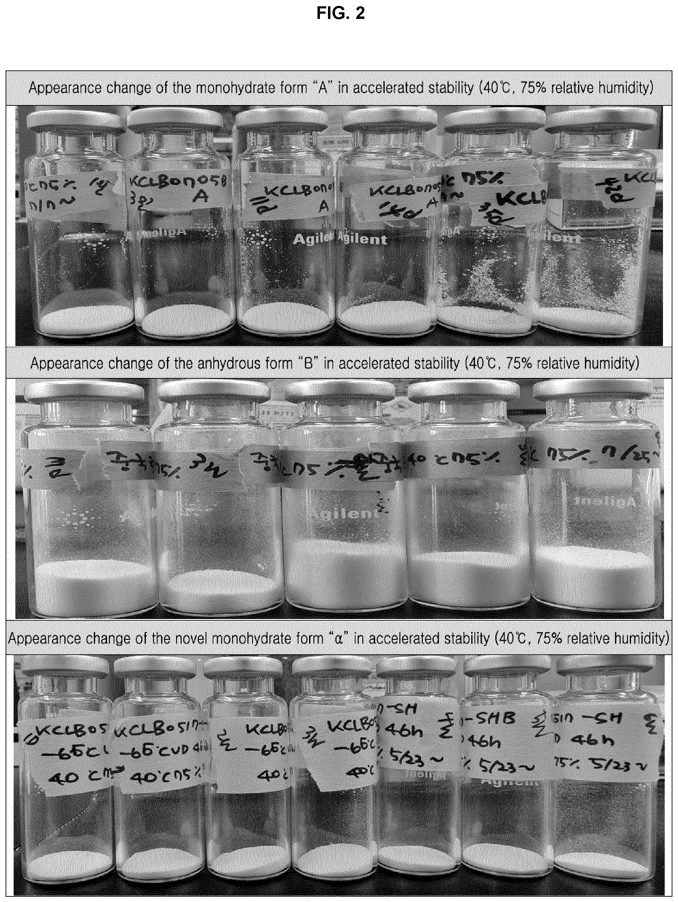

The novel monohydrate form "α" of avibactam sodium salt prepared in Example 1, the monohydrate form "A" and the anhydrous form "B" were each packaged in glass vials and stored at 40°C and 75% relative humidity for 6 weeks and the purity changes thereof were measured by HPLC at weekly intervals. The results of the comparative experiments are shown in FIG. 1 and FIG. 2. It was found therefrom that the novel monohydrate according to the present invention was a more stable form than the monohydrate form "A" described in WO2011/042560 A1.

### Example 3. Accelerated stability test on moisture content changes (40°C, 75% relative humidity)

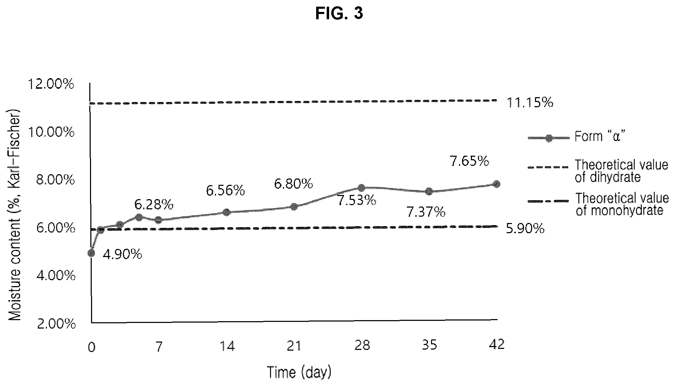

The novel monohydrate form "α" of avibactam sodium salt prepared in Example 1 was packaged in a glass vial and stored at 40°C and 75% relative humidity for 6 weeks and the change in moisture content thereof was measured by a Karl-Fischer moisture meter at weekly intervals for about 6 weeks. The results thereof are shown in FIG. 3. It was confirmed therefrom that the novel monohydrate according to the present invention maintained the monohydrate form thereof in the accelerated stability test (40°C, 75% relative humidity).

### Example 4. XRD pattern measurement

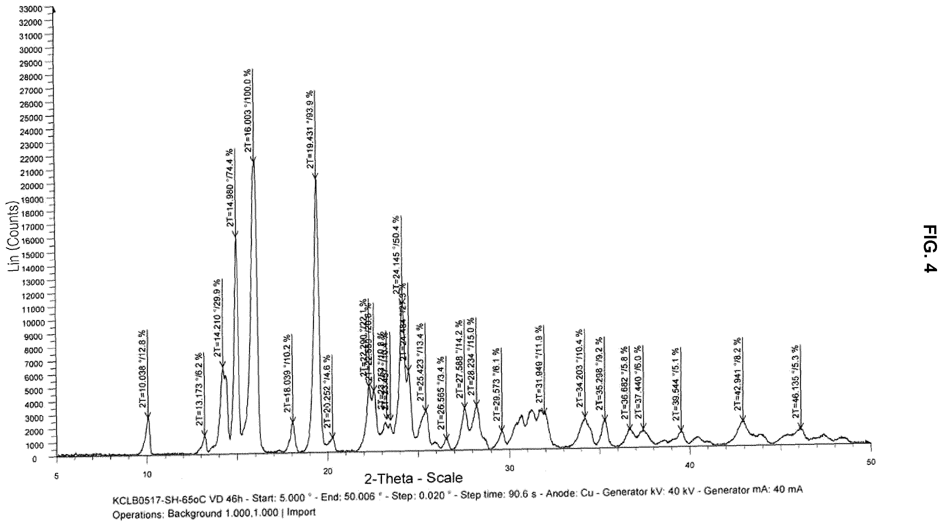

The X-ray powder diffraction of the novel monohydrate form "α" of avibactam sodium salt prepared in Example 1 was confirmed with an equipment having CuKα radiation. The sample was placed on a plate without being crushed and analyzed at an angle 2θ of 5 to 50°, and the results are shown in FIG. 4. It shows different physicochemical properties from the characteristic peaks at 8.5, 15.3, 16.4, 17.0, 24.3 of the monohydrate form "A".

### Example 5. TGA pattern measurement

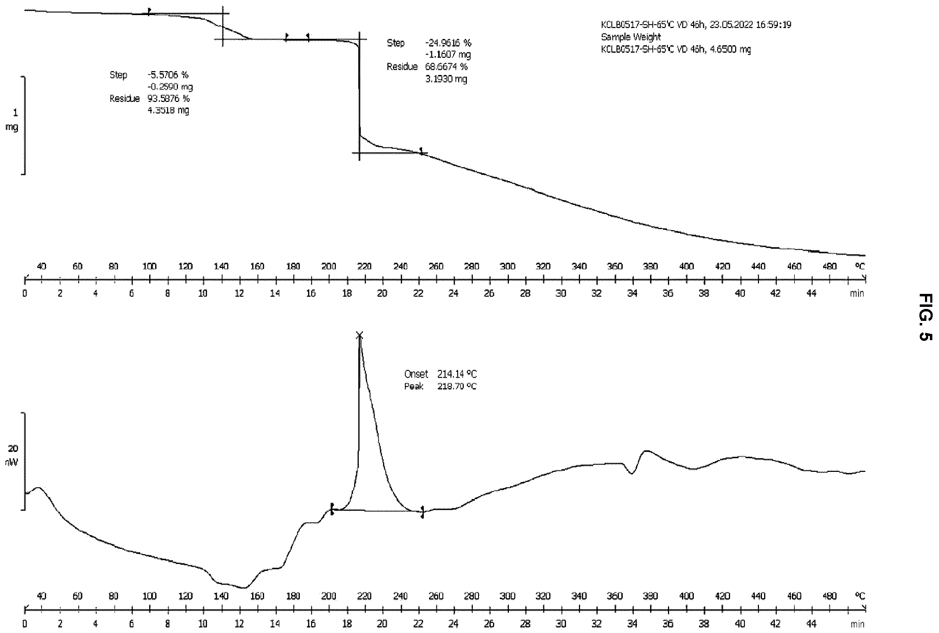

The TGA pattern was measured while heating 3 to 5 mg of the novel monohydrate form "α" of avibactam sodium salt prepared in Example 1 from 30 to 500°C at a rate of 10 K/min. As a result, it was confirmed that the monohydrate showed a weight loss of 5.57% at 100 to 140°C, which corresponds to 5.90% of the theoretical water content of the monohydrate. In addition, decomposition exotherm accompanied by weight loss at 200 to 220°C was confirmed and the results are shown in FIG. 5.

### Example 6. DSC pattern measurement

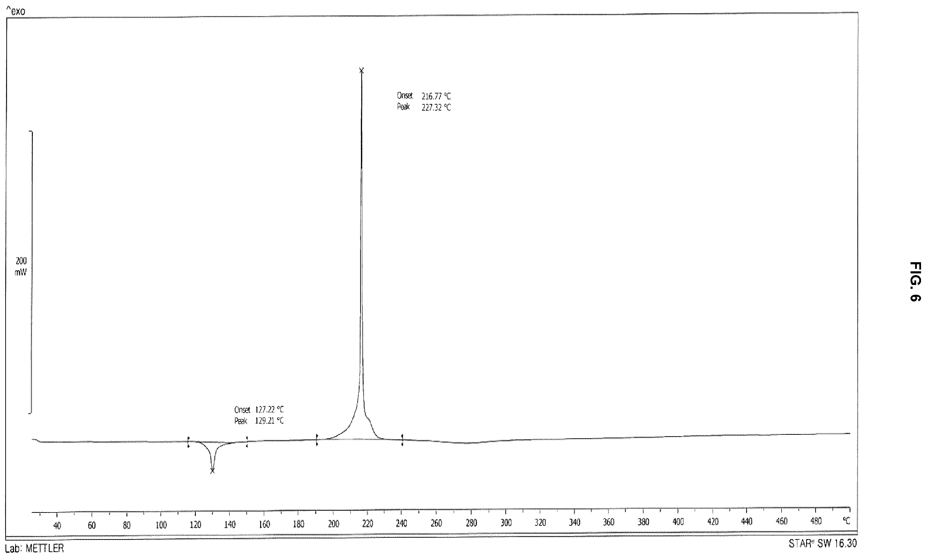

The DSC pattern was measured while heating 3 to 5 mg of the novel monohydrate form "α" of avibactam sodium salt prepared in Example 1 from 25 to 500°C at a rate of 10 K/min. As a result, the exothermic decomposition peak was confirmed at 227.3°C. WO2011/042560 A1 describes that the exothermic decomposition peak of the monohydrate form A is 238.9°C. It was confirmed therefrom that it had different physicochemical properties from the monohydrate form "A", and the results are shown in FIG. 6.

## Claims

1. A crystalline monohydrate of avibactam sodium salt having an X-ray diffraction pattern comprising characteristic peaks at 10.0±0.5°, 15.0±0.5°, 16.0±0.5°, and 19.4±0.5° 2-theta (2θ).

2. The crystalline monohydrate of avibactam sodium salt according to claim 1, having an X-ray diffraction pattern comprising characteristic peaks at 10.0±0.5°, 13.2±0.5°, 14.2±0.5°, 15.0±0.5°, 16.0±0.5°, 18.0±0.5°, and 19.4±0.5° 2-theta (2θ).

3. A process for preparing a crystalline monohydrate of avibactam sodium salt, comprising (a) adding a solution of sodium 2-ethylhexanoate to a solution of avibactam tetrabutylammonium salt; (b) stirring the reaction mixture of step (a) at room temperature, followed by stirring at -10°C to -5°C to form a crystal; and (c) isolating the crystal.

4. The process according to claim 3, which does not comprise seeding a monohydrate of avibactam sodium salt.

5. The process according to claim 3, wherein the solution of avibactam tetrabutylammonium salt is a solution of avibactam tetrabutylammonium salt in a mixed solvent of methanol and water.

6. The process according to claim 5, wherein the mixed solvent of methanol and water is a mixed solvent having a volume ratio of 4 to 8 : 1 of methanol : water.

7. The process according to claim 3, wherein the solution of sodium 2-ethylhexanoate is a solution of sodium 2-ethylhexanoate in methanol or in a mixed solvent of methanol and water.

8. The process according to claim 3, wherein step (c) is carried out by washing the crystal obtained by filtering the mixture of step (b) with acetone at a temperature of 0°C to 5°C, followed by drying the resulting crystal.

9. The process according to claim 3, wherein the crystalline monohydrate of avibactam sodium salt has an X-ray diffraction pattern comprising characteristic peaks at 10.0±0.5°, 15.0±0.5°, 16.0±0.5°, and 19.4±0.5° 2-theta (2θ).

10. The process according to claim 3, wherein the crystalline monohydrate of avibactam sodium salt has an X-ray diffraction pattern comprising characteristic peaks at 10.0±0.5°, 13.2±0.5°, 14.2±0.5°, 15.0±0.5°, 16.0±0.5°, 18.0±0.5°, and 19.4±0.5° 2-theta (2θ).
